# EUROPEAN PATENT SPECIFICATION

(11) **EP 3 930 764 B1**
(45) Date of publication and mention of the grant of the patent: **05.06.2024**
(21) Application number: 20762262.2
(22) Date of filing: 26.02.2020
(51) Int. Cl.: A61K 47/69, A61K 47/60, A61B 5/00

(54) **RENAL CLEARABLE NANOPARTICLES AS EXOGENOUS MARKERS FOR EVALUATING KIDNEY FUNCTION**
RENALE AUSSCHEIDBARE NANOPARTIKEL ALS EXOGENE MARKER ZUR BEURTEILUNG DER NIERENFUNKTION
NANOPARTICULES POUVANT ÊTRE ÉLIMINÉES PAR CLAIRANCE RÉNALE UTILISÉES COMME MARQUEURS EXOGÈNES POUR ÉVALUER LA FONCTION RÉNALE

(30) Priority: 26.02.2019 US 201962810626 P
(43) Date of publication of application: 05.01.2022
(73) Proprietor: Board of Regents, The University of Texas System, Austin, TX 78701 (US)
(72) Inventor: ZHENG, Jie, Plano, TX 75025 (US); YU, Mengxiao, Frisco, TX 75035 (US); NING, Xuhui, Richardson, TX 75080 (US)
(74) Representative: Henderson, Helen Lee
(86) International application number: PCT/US2020/019782
(87) International publication number: WO 2020/176555

(56) References cited:
- WO-A1-2007/028636
- WO-A2-2007/047458
- JIAGUO HUANG ET AL: "Light-Emitting Agents for Noninvasive Assessment of Kidney Function", CHEMISTRY OPEN, vol. 6, no. 4, 20 July 2017 (2017-07-20), pages 456-471, XP055729144, ISSN: 2191-1363, DOI: 10.1002/open.201700065
- XIAO-DONG ZHANG ET AL: "renal clearance, biodistribution, toxicity of gold nanoclusters", BIOMATERIALS, ELSEVIER, AMSTERDAM, NL, vol. 33, no. 18, 5 March 2012 (2012-03-05) , pages 4628-4638, XP028411037, ISSN: 0142-9612, DOI: 10.1016/J.BIOMATERIALS.2012.03.020 [retrieved on 2012-03-07]
- JIANG et al.: "Efficient renal clearance of DNA tetrahedron nanoparticles enables quantitative evaluation of kidney function", Nano Research, vol. 12, no. 3, 28 December 2018 (2018-12-28), pages 637-642, XP036705743, DOI: 10.1007/s12274-019-2271-5

## Description

### GOVERNMENT SUPPORT

This invention was made with government support under Grant No. R43 DK116368 awarded by the National Institutes of Health. The government has certain rights in the invention.

### FIELD OF THE DISCLOSURE

This disclosure relates to the evaluation and diagnosis of kidney disease, such as early detection of kidney dysfunction in living subjects utilizing nanoparticles that can be eliminated from the body by the kidneys as an exogenous marker.

### BACKGROUND

Kidney disease affects more than 10% of the population worldwide (850 million), which is twice the number of people living with diabetes (422 million) and 20 times the number of cancer patients (42 million). Chronic kidney disease (CKD) causes 1.2 million deaths in the world each year, more than breast and prostate cancers combined. In the United States, about 30 million people (15% of adults) are living with CKD that can be caused by diabetes, high blood pressure, glomerulonephritis, cystic kidney disease and other disorders. Different from CKD wherein kidney function is gradually lost over several months or years, acute kidney injury (AKI) is characterized by an abrupt reduction in kidney function within a few hours or days. AKI is a deadly disease and a common clinical complication affecting 13.3 million patients worldwide annually, including 3.2-20% of all hospitalized patients and 22-67% intensive care unit (ICU) patients. Despite supportive care including renal replacement therapy, the 5-year mortality after AKI remains over 50%. In addition, many medicines can induce AKI due to drug-induced nephrotoxicity, such as cisplatin (a chemotherapeutic agent for cancer treatment). Moreover, patients with systemic lupus erythematosus (SLE, lupus, a chronic autoimmune disease) also have a high risk in developing a type of kidney disease - lupus nephritis, a life-threatening complication due to renal deposition of immune complexes. At least 5 million people worldwide and 1.5 million Americans suffer from lupus and over 90% of them are women between the ages of 15-44. While SLE is a systemic disease that affects multiple organs, kidney involvement is a leading cause of morbidity and mortality in SLE: generally, about 60% of lupus patients will develop lupus nephritis during their lives and have a higher mortality than those without kidney involvement.

As a "silent killer," kidney disease often has no signs or symptoms in the early stages and remains undetected until it is very advanced. For instance, approximately 60% of CKD cases get diagnosed only at the end-stage renal disease (ESRD, more than 85% of normal kidney function is lost), which is fatal without dialysis or a kidney transplant. Within 15 years of diagnosis, 10%-30% of lupus nephritis patients progress to ESRD even with treatment. The treatment for ESRD consumes $30.9 billion to care in the United States in 2013, about 7.1 percent of the overall Medicare paid claims costs.

While kidney disease is usually a progressive disease and the decline of kidney function is unavoidable, kidney disease can be managed effectively if it is diagnosed in the early stages before irreversible damages have occurred. For example, drug-induced AKI can often be cured if diagnosed and treated early. For CKD that cannot be cured, early diagnosis and management is crucial not only to delay or prevent progression to ESRD, but also to lower the risk for heart disease and stroke. Therefore, for people who have risk factors for CKD, including diabetes, high blood pressure, heart disease, obesity, and a family history of CKD, it is recommended that kidney function be checked regularly (e.g., every 3 or 6 months) with blood and urine tests. These tests measure endogenous biomarkers such as blood urea nitrogen (BUN), serum creatinine, and urine protein. Lupus nephritis also has no cure. Early diagnosis of kidney involvement and treatment of lupus nephritis is critical to stop kidney damage early so that kidney failure can be postponed or even prevented. Therefore, the kidney function of lupus patients needs to be monitored every 3 months (in average) with blood and urine tests in order to catch lupus nephritis at an early stage. Since proteinuria is more sensitive than serum creatinine in identifying lupus nephritis, once proteinuria is detected, the patients will be subjected to kidney biopsy that is the gold standard for classifying the lupus nephritis and guiding the treatment options.

In addition to early diagnosis of kidney disease, accurate monitoring of kidney function is also necessary during the treatment of kidney disease. For example, lupus nephritis is usually treated with immunosuppressive agents: on one hand, the response to immunosuppressive drugs is quite variable among patients and only -50% of patients responded to treatment in some large trial; on the other hand, immunosuppressive drugs have many potential side effects, such as lowering the blood counts and increasing risks for infection and cancer. Thus, during the treatment of lupus nephritis, doctors need to closely monitor the kidney function of patients (every 1 month in average) so that they can optimize the use of medications to achieve remission of the disease while reducing the side effects. Moreover, monitoring of kidney function can bring awareness to CKD patients on their diets to prevent further damage to the kidneys.

A number of light -emitting agents for noninvasive assessment of kidney function have been described (Huang et al., 2017, Light-Emitting Agents for Noninvasive Assessment of Kidney Function, Chemistry Open, 6:456-471) but none of these previous studies reported that a renal clearable fluorescent agent can detect kidney dysfunction or kidney disease in early stages before elevation of endogenous biomarkers such as blood urea nitrogen (BUN), serum creatinine, and urine protein through a simple blood or urine test. While BSA- and GSH-protected gold nanoclusters have been investigated for renal clearance, biodistribution and toxicity in normal mice, how kidney dysfunction and kidney disease impact their renal clearance remains unknown (Zhang et al., 2012, In vivo renal clearance, biodistribution, toxicity of gold nanoclusters, Biomaterials, 33:4628-4638). There remains a significant challenge in clinical use of noninvasive fluorescence kidney imaging because of limited tissue penetration of the light.

Despite the clinical need, early diagnosis of kidney dysfunction and accurate monitoring of kidney function remain challenging for current methods. Thus, there remains a need in the art for a method for diagnosing the dysfunction of a live kidney in the early stages (or evaluating the function of a live kidney) through a simple urine or blood test that is not only simple, affordable, and widely accessible, but also is highly sensitive and accurate. The present disclosure is provided to address this need with renal clearable nanoparticles and offer advantages not provided by prior diagnostic techniques.

### SUMMARY

The present disclosure provides methods of evaluating or monitoring kidney function of a subject using renal clearable nanoparticles. The invention is as defined in the appended claims.

In one aspect, the present invention provides a method of evaluating kidney function of a subject, the method comprising: (a) characterizing nanoparticles in a urine sample collected from a subject with a measurement process to obtain a characteristic parameter; and (b) comparing the characteristic parameter with a reference value, thereby evaluating the kidney function, wherein a first plurality of nanoparticles having a first dose has been administered to the subject and the urine sample has been collected within a first period of time after the administration.

The first period of time may be about 30 minutes to 24 hours.

In another aspect, the present invention provides a method of evaluating kidney function of a subject, the method comprising: (a) characterizing nanoparticles in a blood sample collected from a subject, with a measurement process to obtain a characteristic parameter; and (b) comparing the characteristic parameter with a reference value, thereby evaluating the kidney function, wherein a first plurality of nanoparticles having a first dose has been administered to the subject and the blood sample has been collected within a first period of time after the administration.

The first period of time may be about 5 mins to 1 hour.

The reference value may be a control characteristic parameter measured for a control group having normal kidney function.

The method may further comprise indicating kidney dysfunction or injury when the characteristic parameter is significantly different from the control characteristic parameter or reference value.

The kidney dysfunction may be caused by drug-induced nephrotoxicity, an autoimmune disease, kidney failure, chronic kidney disease, cystic kidney disease, kidney inflammatory disease, kidney fibrosis, autosomal dominant polycystic kidney disease, an immuno-oncological treatment, an infection or a combination thereof, preferably wherein the autoimmune disease is lupus or wherein the kidney inflammatory disease is lupus nephritis.

In yet another aspect, the present invention provides a method of monitoring kidney function of a subject, the method comprising: (a) characterizing nanoparticles in a first urine sample with a measurement process to obtain a first characteristic parameter; (b) characterizing nanoparticles in a second urine sample with the measurement process to obtain a second characteristic parameter; and (c) comparing the first characteristic parameter with the second characteristic parameter, thereby monitoring the kidney function over time, wherein a first plurality of nanoparticles having a first dose has been administered to the subject and the first urine sample has been collected within a first period of time after the administration of the first plurality of nanoparticles; and wherein a second plurality of nanoparticles having the first dose has been administered to the subject after a second period of time and the second urine sample has been collected within a third period of time after the administration of the second plurality of nanoparticles.

In yet another aspect, the present invention provides a method of monitoring kidney function of a subject, the method comprising: (a) characterizing a first plurality of nanoparticles in a first blood sample collected within a first period of time from the subject administered with the first plurality of nanoparticles, with a measurement process to obtain a first characteristic parameter; (b) after a second period of time after step (a), characterizing a second plurality of nanoparticles in a second blood sample collected after a third period of time from the subject administered with the second plurality of nanoparticles, with the measurement process to obtain a second characteristic parameter; and (c) comparing the first characteristic parameter with the second characteristic parameter, thereby monitoring the kidney function over time.

The second period of time may be about 1 hour to 30 days for acute kidney disease, or wherein the second period of time is about two weeks to 12 months for chronic kidney disease.

The nanoparticles may be renal clearable.

Optionally the nanoparticles have a 1-hour or 2-hour renal clearance efficiency in the range of 5 to 100 percent of injected dose (% ID); or the nanoparticles comprise gold, silver, copper, platinum, palladium, silica, carbon, silicon, iron oxide, FeS, CdSe, CdS, CuS, an organic material, or a combination thereof.

The nanoparticles may be coated with a ligand selected from the group consisting of glutathione, thiol-functionalized polyethylene glycol, cysteamine, cysteine, homocysteine, a dipeptide containing cysteine, a dipeptide containing homocysteine, a peptide having more than three amino acids, and a combination thereof.

Optionally, the dipeptide containing cysteine includes cysteine-glycine or cysteine-glutamic acid; or wherein the dipeptide containing homocysteine includes homocysteine-glycine or homocysteine-glutamic acid; or wherein the ligand is conjugated with a fluorescent dye; or wherein the ligand is glutathione.

Optionally, the nanoparticles fluoresce in a range of 500 to 850 nm; or the nanoparticles fluoresce in a range of 1000 to 1700 nm.

Optionally the first period of time is about 30 minutes to 24 hours for the urine sample; or the third period of time is about 30 minutes to 24 hours for the urine sample.

The measurement process may include one or more processes selected from the group consisting of: measuring a concentration of the nanoparticles, measuring an amount of the nanoparticles, measuring clearance efficiency, analyzing a composition of ligands on the nanoparticle surface, measuring size distribution of the nanoparticles, measuring an absorption spectrum of the nanoparticles, measuring an emission spectrum of the nanoparticles, measuring an excitation spectrum of the nanoparticles, measuring a photoacoustic signal of the nanoparticles, measuring radioactivity of the nanoparticles, or a combination thereof, preferably wherein the measurement process includes inductively coupled plasma mass spectrometry (ICP-MS) or inductively coupled plasma optical emission spectroscopy (ICP-OES).

The characteristic parameter may be selected from the group consisting of: a concentration of the nanoparticles, an amount of the nanoparticles, clearance efficiency, a composition of ligands on the nanoparticle surface, one type of surface ligand, size distribution of the nanoparticles, surface charge of the nanoparticles, an absorption spectrum of the nanoparticles, an emission spectrum of the nanoparticles, an excitation spectrum of the nanoparticles, a photoacoustic signal of the nanoparticles, radioactivity of the nanoparticles, and a combination thereof.

Optionally, ai) the characteristic parameter is the concentration of the nanoparticles in the blood sample of the subject; (aii) the control characteristic parameter is the control concentration of the nanoparticles in the blood sample of the control group; and (aii) if the concentration is significantly different from the control concentration, then it indicates kidney dysfunction or injury; or (bi) the characteristic parameter is the clearance efficiency of the nanoparticles in the subject; (bii) the control characteristic parameter is the control clearance efficiency of the nanoparticles in the control group; and (biii) if the clearance efficiency is significantly different from the control clearance efficiency, then it indicates kidney dysfunction or injury; or (ci) the characteristic parameter is the emission spectrum of the nanoparticles in the urine sample of the subject; (cii) the control characteristic parameter is the control emission spectrum of the nanoparticles in the urine sample of the control group; and (ciii) if the emission spectrum is significantly different from the control emission spectrum, then it indicates kidney dysfunction or injury.

Optionally, the administration is to be intravenous, intraperitoneal, subcutaneous, or intraarterial.

### BRIEF DESCRIPTION OF THE DRAWINGS

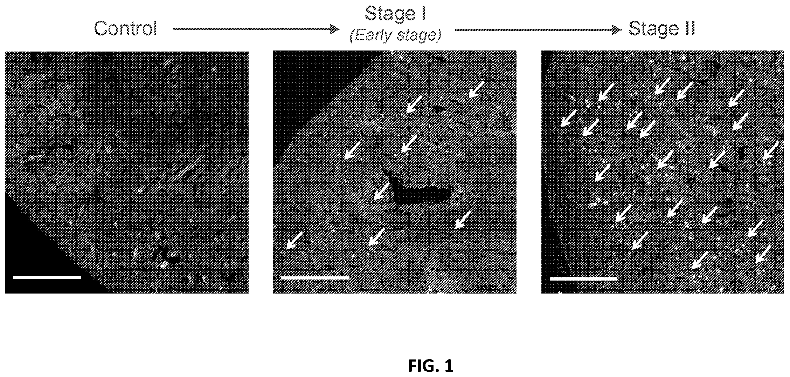

**FIG. 1** is a series of fluorescence images of terminal deoxynucleotidyl transferase dUTP nick end labeling (TUNEL) assay analysis of kidney tissues, which were obtained from mice receiving cisplatin (15 mg/kg, intraperitoneal injection) or phosphate buffered saline ("Control"). The TUNEL assay analysis revealed two different levels of cell apoptosis in the kidney, corresponding to two different stages of cisplatin-induced acute kidney injury. The apoptotic cells, labeled by FITC, were pointed by arrows. The nuclei were stained by DAPI. Stage I was an "early stage" where apoptosis was identified in the kidney tissues of cisplatin-injected mice, in contrast to the negligible apoptosis in the normal kidneys of control group. Stage II showed significantly enhanced cell apoptosis than Stage II. Scale bar, 500 µm for all the three images.

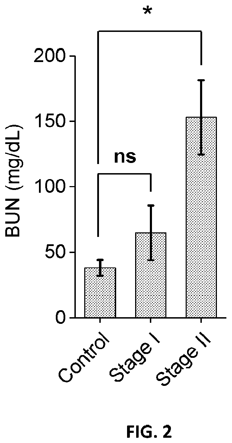

**FIG. 2** is a graph showing the levels of blood urea nitrogen (BUN) of mice in two different stages of cisplatin-induced kidney injury and control group having normal kidney function. Mice receiving phosphate buffered saline served as control. N=4 for each group, *P<0.05; ns, no significant difference, P>0.05.

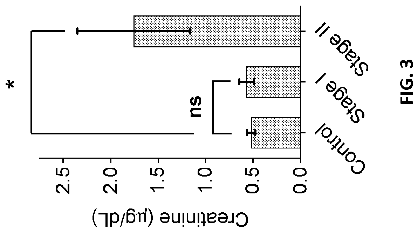

**FIG. 3** is a graph showing the levels of serum creatinine of mice in two different stages of cisplatin-induced kidney injury and control group having normal kidney function. Mice receiving phosphate buffered saline served as control. N=4 for each group, *P<0.05; ns, no significant difference, P>0.05.

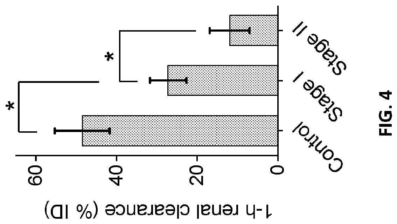

**FIG. 4** is a graph showing the amount of renal clearable glutathione-coated gold nanoparticles (GS-AuNPs) that were excreted in urine within 1 hour post intravenous injection (namely "1-h renal clearance"). Mice in two different stages of cisplatin-induced kidney injury were studied. Mice receiving phosphate buffered saline served as control. % ID, percent of injected dose; N=4 for each group, *P<0.05.

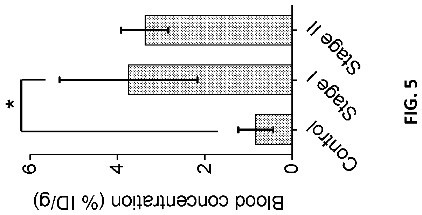

**FIG. 5** is a graph showing the blood concentration of renal clearable glutathione-coated gold nanoparticles (GS-AuNPs) at 1 hour post intravenous injection. Mice in two different stages of cisplatin-induced kidney injury were studied. Mice receiving phosphate buffered saline served as control. % ID/g, percent of injected dose per gram of blood; N=4 for each group, *P<0.05.

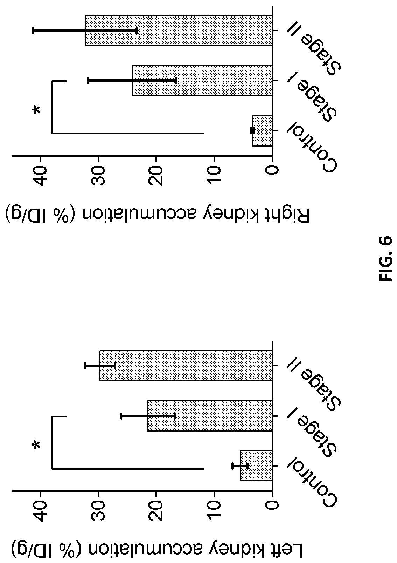

**FIG. 6** are graphs showing the amount of renal clearable glutathione-coated gold nanoparticles (GS-AuNPs) in the left and right kidneys at 1 hour post intravenous injection. Mice in two different stages of cisplatin-induced kidney injury were studied. Mice receiving phosphate buffered saline served as control. % ID/g, percent of injected dose per gram of blood; N=4 for each group, *P<0.05.

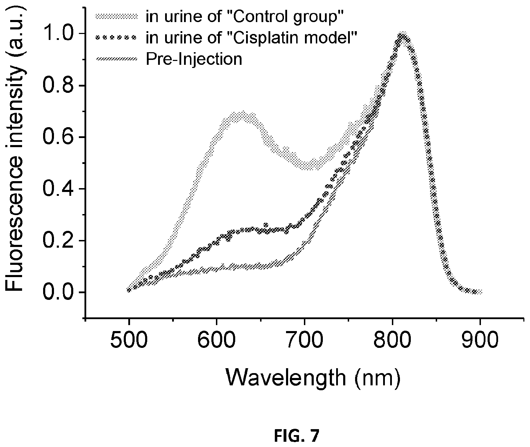

**FIG. 7** is a graph showing the representative emission spectra of GS-AuNPs before being injected ("Pre-Injection") and being excreted into urine of mice. Control group and cisplatin model were studied.

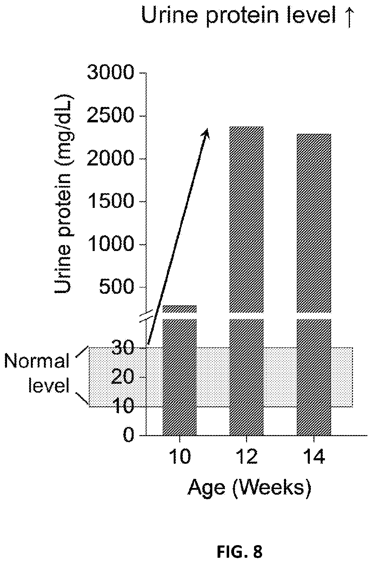

**FIG. 8** is a graph showing the changes of urine protein levels of an MRL-lpr lupus mouse during growth from age of 10 to 14 weeks. Normal level values were obtained from four control MRL mice at age of 8 weeks.

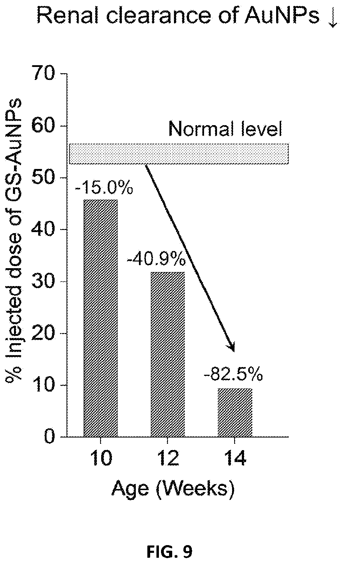

**FIG. 9** is a graph showing the changes of 1-hour renal clearance efficiencies of GS-AuNPs (5 mg/kg, after intravenous injection) of an MRL-lpr lupus mouse during growth from age of 10 to 14 weeks. The changes of urine protein levels of this lupus mouse are shown in FIG. 8. Normal level values were obtained from four control MRL mice at age of 8 weeks.

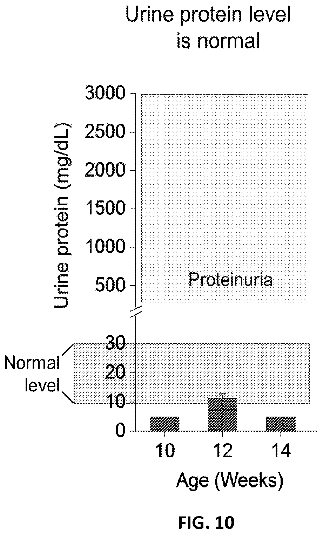

**FIG. 10** is a graph showing the urine protein levels of three MRL-lpr lupus mice during growth from age of 10-14 weeks. Normal level values were obtained from control MRL mice at age of 8 weeks. *P< 0.05, T test.

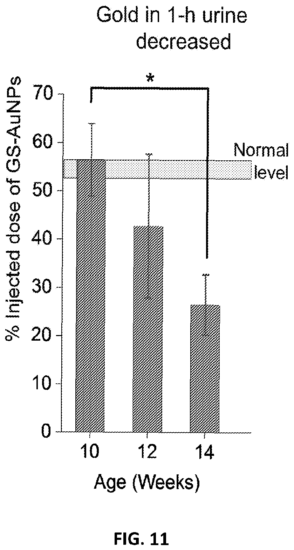

**FIG. 11** is a graph showing the 1-hour renal clearance efficiencies of 5 mg/kg GS-AuNPs after intravenous injection of three MRL-lpr lupus mice during growth from age of 10-14 weeks. The changes of urine protein levels of these lupus mouse are shown in FIG. 10. Normal level values were obtained from control MRL mice at age of 8 weeks. *P< 0.05, T test.

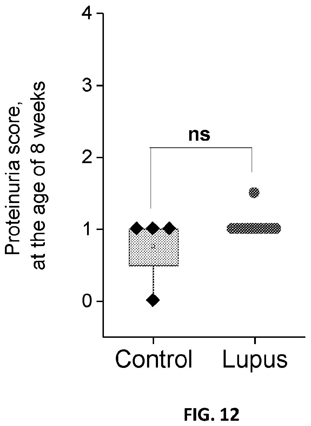

**FIG. 12** is a graph showing the levels of urine protein of lupus model and control group at the age of 8 weeks. Lupus model was MRL/MpJ-Faslpr/J mouse (also known as MRL-lpr), a well-established model of systemic lupus erythematosus (SLE). Control group was MRL/MpJ mouse, also known as MRL. Left, proteinuria score of the urine samples was measured using Albustix^{®} reagent strips for analysis of urine protein. Grades of proteinuria were scored as follows: 0 = none, 1 = trace, 1.5 = trace-30 mg/dl, 2 = 30 mg/dl, 3 = 100 mg/dl, 4 = 300 mg/dl, and 5 = >2,000 mg/dl. N=11 for lupus model (urine collection for the tests was failed in one mouse); N=4 for control mice; ns, no significant difference, P>0.05.

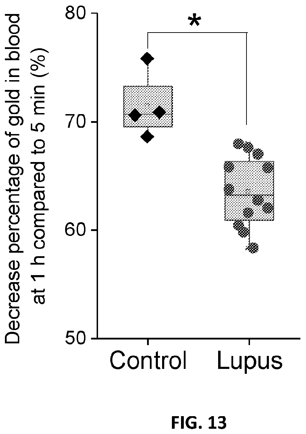

**FIG. 13** is a graph showing the difference between lupus model and control group at the age of 8 weeks in the decrease percentage of blood concentration of renal clearable GS-AuNPs at 1 h compared to that at 5 min (=[(blood concentration at 5 min - blood concentration at 1 h)/ blood concentration at 5 min] x 100%). N=12 for lupus model; N=4 for control mice; *P<0.05.

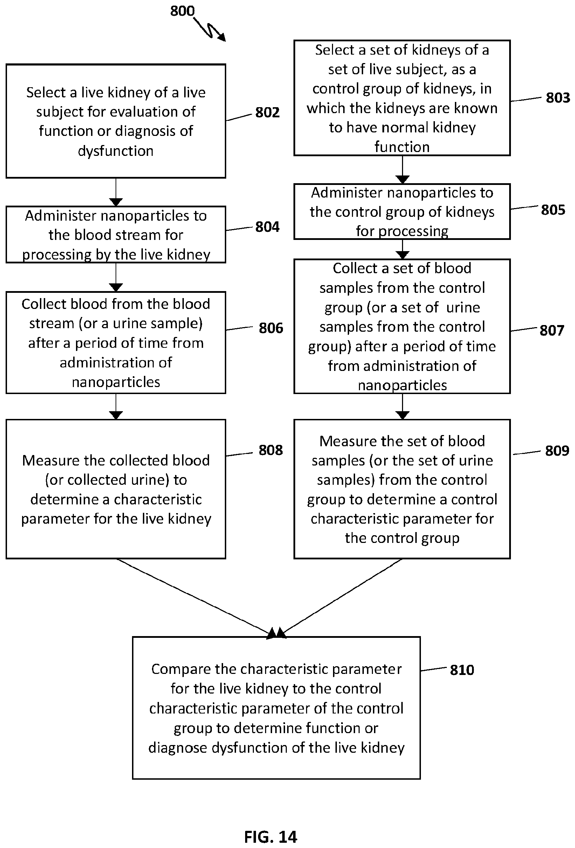

**FIG. 14** is a flow diagram of a method for evaluating the function of a live kidney, the method being further applicable to diagnose a dysfunction of a live kidney.

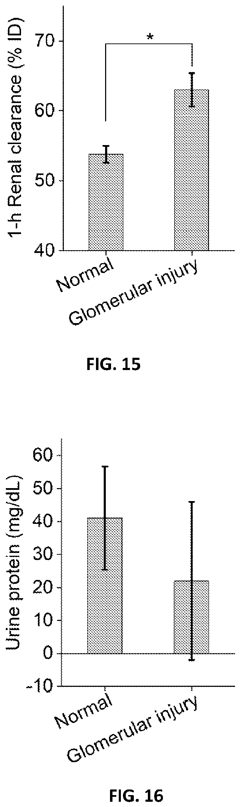

**FIG. 15** is a graph showing the amount of renal clearable glutathione-coated gold nanoparticles (GS-AuNPs) that were excreted in urine of normal mice and mice with glomerular injury within 1 hour post intravenous injection (namely "1-h renal clearance"). % ID, percent of injected dose; N=4 for control group, N=2 for glomerular injury, *P<0.05.
**FIG. 16** are graphs showing the urine protein levels of normal mice and mice with glomerular injury. % ID, percent of injected dose; N=4 for control group, N=2 for glomerular injury.

### DETAILED DESCRIPTION

The present disclosure relates to methods that utilize renal clearable nanoparticles to evaluate and/or monitor kidney function in a subject. After the renal clearable nanoparticles have been administered to a subject, at least a portion of the nanoparticles in the bloodstream is removed by the kidney as part of the urine. These nanoparticles, in a blood sample or a urine sample, can produce one or more quantifiable characteristic parameters, which can be significantly different in a subject with normal kidney function versus a subject with kidney dysfunction or kidney injury. Therefore, these nanoparticles can be used as an exogenous marker to indicate the health of the kidney. Notably, the methods described herein can detect kidney dysfunction or injury even in situations where current serum or urine biomarkers fail to do so, thereby permitting early detection of kidney dysfunction or injury. For the avoidance of doubt, any references to methods including steps such as "obtaining samples from a patient" and/or to administering substances or compositions to a subject in this Detailed Description and the below Examples are to be interpreted as references to compounds, pharmaceutical compositions and medicaments of the present invention for use in those methods.

The nanoparticles can be made of one or more materials. Optionally, the nanoparticles can comprise gold, silver, copper, platinum, palladium, silica, carbon, silicon, iron oxide, FeS, a semiconductor quantum dot, an organic material, or a combination thereof. Examples of semiconductor quantum dots include, but are not limited to, CdTe, CdSe, CdS, and CuS.

The nanoparticles can comprise an organic material that can be eliminated through the kidneys and have sizes in the range of about 0.3 nm to 10 nm or a molecular weight in the range of 500 to 20,000 Dalton. The organic nanoparticles can be labeled with dyes or other probes.

The nanoparticles can comprise an organic material with an average size below about 8 nm, such as PEGylated organic dyes and zwitterionic organic dyes.

The nanoparticles can comprise a core and optionally a surface coating surrounding the core. Optionally, the nanoparticles have an average core size of no more than about 6 nm, no more than about 5.5 nm, no more than about 5 nm, no more than about 4.5 nm, no more than about 4 nm, no more than about 3.5 nm, no more than about 3 nm, or no more than about 2.5 nm. The nanoparticles may have an average core size of at least about 0.5 nm, at least about 0.8 nm, or at least about 1 nm.

Combinations of the above-referenced ranges for the average core size are also possible (*e.g.,* at least about 0.5 nm to no more than about 6 nm, or at least about 0.5 nm to no more than about 2.5 nm), inclusive of all values and ranges therebetween. The nanoparticles may be gold nanoparticles having an average core size of at least about 0.5 nm to no more than about 2.5 nm.

Optionally, the nanoparticles have an average hydrodynamic diameter of no more than about 10 nm, no more than about 9 nm, no more than about 8 nm, no more than about 7 nm, no more than about 6 nm, no more than about 5 nm, or no more than about 4 nm. The nanoparticles may have an average hydrodynamic diameter of at least about 1 nm, at least about 1.5 nm, or at least about 2 nm, at least about 2.5 nm.

Combinations of the above-referenced ranges for the average hydrodynamic diameter are also possible (*e.g*., at least about 1 nm to no more than about 10 nm, or at least about 1 nm to no more than about 4 nm), inclusive of all values and ranges therebetween.

Optionally, the core of each nanoparticle can comprise no more than about 41,000 atoms, no more than about 35,000 atoms, no more than about 30,000 atoms, no more than about 25,000 atoms, or no more than about 9,000 atoms. The core of each nanoparticle may comprise at least about 2 atoms, at least about 25 atoms, at least about 30 atoms, at least about 35 atoms, or at least about 40 atoms.

Combinations of the above-referenced ranges for the number of atoms for the core of each nanoparticle are also possible (*e.g*., at least about 2 to no more than about 41,000, or at least about 25 to no more than about 35,000), inclusive of all values and ranges therebetween. Optionally, the nanoparticles comprise between 10 and 650 metal atoms with an average core size of between 0.5 nm and 2.5 nm.

The surface coating can comprise one or more types of ligands. Optionally, the ligand can be selected from the group consisting of glutathione, thiol-functionalized polyethylene glycol (PEG), cysteamine, cysteine, homocysteine, a dipeptide containing cysteine, a dipeptide containing homocysteine, a peptide having more than three amino acids, and a combination thereof. The dipeptide containing cysteine may include cysteine-glycine or cysteine-glutamic acid. the dipeptide containing homocysteine may include homocysteine-glycine or homocysteine-glutamic acid.

Optionally, the thiol-functionalized PEG has a molecular weight in the range of about 150 to 10,000 Dalton.

The ligand can be conjugated with a fluorescent dye.

The nanoparticles can fluoresce on their own or due to the fluorescent dye. The nanoparticles may fluoresce in the visible range, *e.g.,* in a range of 500 to 850 nm. Alternatively, the nanoparticles may fluoresce in the near-infrared range, *e.g.,* in a range of 1000 to 1700 nm.

The nanoparticles can be in the form of an aqueous solution or suspension. The aqueous solution or suspension can further include an agent for preventing the nanoparticles from forming aggregates.

In one aspect, the present disclosure provides a method of evaluating kidney function of a subject, the method comprising: (a) characterizing nanoparticles in a urine sample collected from a subject with a measurement process to obtain a characteristic parameter; and (b) comparing the characteristic parameter with a reference value, thereby evaluating the kidney function, wherein a first plurality of nanoparticles having a first dose has been administered to the subject and the urine sample has been collected within a first period of time after the administration.

In another aspect, the present disclosure provides a method of evaluating kidney function of a subject, the method comprising: (a) characterizing a first plurality of nanoparticles in a blood sample collected from a subject, with a measurement process to obtain a characteristic parameter; and (b) comparing the characteristic parameter with a reference value, thereby evaluating the kidney function, wherein a first plurality of nanoparticles having a first dose has been administered to the subject and the blood sample has been collected within a first period of time after the administration.

A control characteristic parameter can be measured by: (e) administering to a control group a second plurality of the nanoparticles having a second dose; (f) collecting a urine sample and/or a blood sample from the control group after the first period of time after the administration; and (g) characterizing the nanoparticles in the urine sample and/or the blood sample with the measurement process to obtain the control characteristic parameter.

If the characteristic parameter is measured in a blood sample of the subject, then for comparison purposes, the control characteristic parameter is also measured in one or more blood samples of the control group. Similarly, if the characteristic parameter is measured in a urine sample of the subject, then for comparison purposes, the control characteristic parameter is also measured in one or more urine samples of the control group.

The control group can comprise one or more subjects with normal kidney function. The control group can comprise about 5 to 20, about 20 to 100, about 100 to 250, about 250 to 500, about 500 to 1,000, or a combination thereof, or more than 1,000 subjects with normal kidney function.

The administration may be intravenous, intraperitoneal, subcutaneous, or intraarterial. The nanoparticles may be injected intravenously.

The dose (*e.g*., a first dose, a second dose) can depend on the type of nanoparticles and/or the technique used in obtaining the characteristic parameter. For example, for inductively coupled plasma mass spectroscopy, the dose can be in the range of about 10⁻⁹ mmol/kg to about 10⁻³ mmol/kg; for magnetic resonance imaging, the dose can be in the range of about 0.01 mmol/kg to about 10 mmol/kg; for radioactive detection, the dose can be in the range of about 10⁻¹¹ mmol/kg to about 10⁻⁶ mmol/kg.

The first dose may be the same as the second dose. Alternatively, the first dose may be different from the second dose.

After the nanoparticles are administered to a subject, they enter the bloodstream of the subject. After a certain period of time, at least a portion of the nanoparticles in the bloodstream is removed by the kidney as part of the urine. Kidney removal of the nanoparticles may be through glomerular filtration, renal tubular reabsorption, renal tubular secretion, or combinations thereof.

The nanoparticles can interact with the blood, kidney, and/or urine of a subject with normal kidney function in a manner different from that of a subject with kidney dysfunction or injury, thereby resulting in a significant difference in the characteristic parameter. Accordingly, the method may further comprise indicating kidney dysfunction or injury when the characteristic parameter is significantly different from the control characteristic parameter or reference value. The kidney dysfunction or injury may be early stage. Alternatively, the kidney dysfunction or injury may be late stage.

The measurement process used to obtain the characteristic parameter or control characteristic parameter includes one or more processes selected from the group consisting of: measuring a concentration of the nanoparticles, measuring an amount of the nanoparticles, measuring clearance efficiency, analyzing a composition of ligands on the nanoparticle surface, measuring size distribution of the nanoparticles, measuring an absorption spectrum of the nanoparticles, measuring an emission spectrum of the nanoparticles, measuring an excitation spectrum of the nanoparticles, measuring a photoacoustic signal of the nanoparticles, measuring radioactivity of the nanoparticles, or a combination thereof.

The measurement process may include inductively coupled plasma mass spectrometry (ICP-MS). The measurement process may include inductively coupled plasma optical emission spectroscopy (ICP-OES).

Related to the measurement process, the characteristic parameter or control characteristic parameter can be selected from the group consisting of: a concentration of the nanoparticles, an amount of the nanoparticles, clearance efficiency, a composition of ligands on the nanoparticle surface, one type of surface ligand, size distribution of the nanoparticles, surface charge of the nanoparticles, an absorption spectrum of the nanoparticles, an emission spectrum of the nanoparticles, an excitation spectrum of the nanoparticles, a photoacoustic signal of the nanoparticles, radioactivity of the nanoparticles, and a combination thereof.

When there is kidney dysfunction or injury, the clearance efficiency of the nanoparticles in the urine is significantly different as compared to a control with normal kidney function. For example, see FIG. 4 and FIG. 15. Without wishing to be bound by theory, this is because the clearance efficiency depends on the mechanism of kidney dysfunction or injury. Accordingly, in one example, the characteristic parameter is the clearance efficiency of the nanoparticles in the urine sample of the subject; the control characteristic parameter is the control clearance efficiency of the nanoparticles in the urine sample of the control group; and if the clearance efficiency is significantly different from the control clearance efficiency, then it indicates kidney dysfunction or injury.

Optionally, the clearance efficiency is significantly less than the control clearance efficiency, *e.g.,* the clearance efficiency is about 95%, about 90%, about 85%, about 80%, about 75%, about 70%, about 65%, about 60%, about 55%, about 50%, about 45%, about 40%, about 35%, about 30%, about 25%, about 20%, about 15%, or about 10% of the control clearance efficiency.

Optionally, the clearance efficiency is significantly greater than the control clearance efficiency, *e.g.,* the clearance efficiency is about 105%, about 110%, about 115%, about 120%, about 125%, about 130%, about 135%, about 140%, about 145%, about 150%, about 155%, about 160%, about 165%, about 170%, about 175%, about 180%, about 185%, or about 190% of the control clearance efficiency.

When there is kidney dysfunction or injury, the concentration of the nanoparticles in the blood is significantly different as compared to a control with normal kidney function. For example, see FIG. 5. Accordingly, in one example, the characteristic parameter is the concentration of the nanoparticles in the blood sample of the subject; the control characteristic parameter is the control concentration of the nanoparticles in the blood sample of the control group; and if the concentration is significantly different from the control concentration, then it indicates kidney dysfunction or injury.

The concentration of the nanoparticles may be significantly less than the control concentration, *e.g*., the concentration of the nanoparticles is about 95%, about 90%, about 85%, about 80%, about 75%, about 70%, about 65%, about 60%, about 55%, about 50%, about 45%, about 40%, about 35%, about 30%, about 25%, about 20%, about 15%, or about 10% of the control concentration.

The concentration of the nanoparticles may be significantly greater than the control concentration, *e.g*., the concentration of the nanoparticles is about 105%, about 110%, about 115%, about 120%, about 125%, about 130%, about 135%, about 140%, about 145%, about 150%, about 155%, about 160%, about 165%, about 170%, about 175%, about 180%, about 185%, or about 190% of the control concentration.

During circulation in the bloodstream, the nanoparticle surface may acquire a different amount of biological thiols in the blood of a subject with kidney dysfunction or injury as compared to a control with normal kidney function. As a result, the emission spectrum of the nanoparticles excreted from a dysfunctional or injured kidney can be significantly different from that of the nanoparticles excreted from a kidney with normal function.

Alternatively, instead of relying on a control characteristic parameter, a reference value can be used for comparison purposes. The reference value can be obtained from a database, which includes the characteristic parameters of controls having normal kidney function and statistical averages thereof. The statistical average may be an arithmetic mean, a geometric mean, or a harmonic mean. Optionally, the reference value can be a range.

Referring to FIG. 14, an example of a method **800,** for evaluating kidney function is shown. The method shows at step **802** selection of a live kidney from a live subject for evaluation or diagnosis and then continues, at step **804,** shows administration of nanoparticles to the blood stream of the live subject via a blood vessel connected to the live kidney so that the live kidney processes the blood stream. Step **806,** shows collection of a blood sample from the blood stream (processed by the live kidney) after a first period of time. Alternatively, step **806** shows collection of a urine sample (processed by the live kidney) from the live subject in a second period of time. The period of time for collecting blood or for collecting urine can be the same or different.

After collection, at step **808,** the blood sample or the urine sample are analyzed using an appropriate measurement process to obtain a characteristic parameter for the live kidney under test. A control characteristic parameter may be measured for a control group of kidneys known to have normal kidney function as shown in steps **803, 805, 807** and **809.**

Then, at step **810,** the measured characteristic parameter is compared to the control characteristic parameter to evaluate and determine the function (or diagnose the dysfunction or injury) of the live kidney.

It should be understood that for the control group, a set of kidneys, known to be functioning normally may be selected (step **803**)**.** Then, at step **805,** nanoparticles can be administered to a blood vessel connected to at least one kidney of the control group. At step **807,** at least one of: a blood sample or a urine sample can be collected after the first period of time after administration of the nanoparticles (the same first period of time as in step **806**) or collecting total urine sample within the second period of time after administration of the nanoparticles (the same second period of time as in step **806**), wherein the blood sample and the urine sample have been processed by the at least one kidney. At step **809,** the nanoparticles in the urine sample and the blood sample are characterized using the same type of measurement process as in step **808** to obtain the control characteristic parameter for the at least one kidney.

Optionally, the method **800** may begin at step **808,** while steps **802, 804,** and **806** may be conducted separately from step **808** and or step **810,** which may be carried out by a medical professional, such as a doctor or a nurse, or the subject. Similarly, steps **803, 805,** and **807** may be conducted separately from step **809** and/or step **810,** which may be carried out by a medical professional, such as a doctor or a nurse, or the subject.

For the avoidance of doubt, steps **802** to **807** are described herein for illustrative purposes only and are not encompassed by the wording of the claims.

Method **800** as applied to diagnose the dysfunction or injury of a live kidney is especially sensitive to detecting early stages of kidney dysfunction when renal function biomarkers such as BUN, serum creatinine and urine proteins remain in the normal range. The dysfunction of the live kidney is not limited to acute kidney disease such as drug-induced nephrotoxicity. The methods described herein are also applicable when the dysfunction of the kidney is caused by at least one of: an autoimmune disease such as lupus; chronic kidney disease that is related to diabetes, glomerulonephritis, or high blood pressure; kidney failure; kidney inflammatory disease, such as lupus nephritis; kidney fibrosis; autosomal dominant polycystic kidney disease; an immuno-oncological treatment; cystic kidney disease; or a combination thereof.

The immuno-oncological treatment may be an immunotherapy.

The kidney injury may be induced by an infection, *e.g.,* a viral infection, a bacterial infection, or a parasite infection.

The kidney injury may be caused by ureter injury and other related ureter trauma, such as ureteral obstruction and ureteral discontinuity.

Further to the measurement process, blood collection may be performed at one time point (such as 1 hour or 24 hours post administration), at two time points (such as 5 minutes and 1 hour post administration), or at multiple time points (such as 5 minutes, 1 hour, and 24 hours post administration). Optionally, the blood sample is collected about 5 minutes to 1 hour post administration.

Further to the measurement process, the urine sample is collected within a period of time, such as about 30 minutes to 1 hour or about 30 minutes to 24 hours post administration.

The urine sample may be collected from the ureter of a donor kidney for transplant.

In the step of comparing, statistical analysis is applied to determine whether there are statistically significant differences between the two data sets. A statistically significant difference in the characteristic parameter (for the live kidney under test), when compared to the control characteristic parameter, indicates a renal dysfunction or injury.

The nanoparticles described herein can also be used to monitor over time the kidney function of a subject-particularly a subject with or susceptible to kidney dysfunction or injury.

Accordingly, in one aspect, the present disclosure provides a method of monitoring kidney function of a subject, the method comprising: (a) characterizing nanoparticles in a first urine sample with a measurement process to obtain a first characteristic parameter; (b) characterizing nanoparticles in a second urine sample with the measurement process to obtain a second characteristic parameter; and (c) comparing the first characteristic parameter with the second characteristic parameter, thereby monitoring the kidney function over time; wherein a first plurality of nanoparticles having a first dose has been administered to the subject and the first urine sample has been collected within a first period of time after the administration of the first plurality of nanoparticles; and wherein a second plurality of nanoparticles having the first dose has been administered to the subject after a second period of time and the second urine sample has been collected within a third period of time after the administration of the second plurality of nanoparticles.

The first dose may be the same as the second dose. Alternatively, the first dose is different from the second dose.

In yet another aspect, the present disclosure provides a method of monitoring kidney function of a subject, the method comprising: (a) characterizing a first plurality of nanoparticles in a first blood sample within a first period of time from the subject administered with the first plurality of nanoparticles, with a measurement process to obtain a first characteristic parameter; (b) after a second period of time after step (a), characterizing a second plurality of nanoparticles in a second blood sample collected after a third period of time from the subject administered with the second plurality of nanoparticles, with the measurement process to obtain a second characteristic parameter; and (c) comparing the first characteristic parameter with the second characteristic parameter, thereby monitoring the kidney function over time.

The third period of time may be the same as the first period of time. Alternatively, the third period of time is different from the first period of time.

Optionally, the first period of time is about 30 minutes to 2 hours for the urine sample, or about 5 mins to 1 hour for the blood sample.

The third period of time may be about 30 minutes to 24 hours for the urine sample, or about 5 mins to 24 hours for the blood sample.

The second period of time and the frequency of monitoring depend on the severity of the kidney dysfunction or injury. For acute kidney disease, the second period of time can be about 1 hour to several weeks, *e.g.,* about 1 hour to 30 days, about 1 hour to 4 weeks, about 1 hour to 3 weeks, about 1 hour to 14 days, about 1 hour to 7 days, about 4 hours to 30 days, about 4 hours to 14 days, or about 4 hours to 7 days. For chronic kidney disease, the second period of time can be about several weeks to several months, *e.g*., about 2 weeks to 12 months, about 2 weeks to 6 months, about 2 weeks to 5 months, about 2 weeks to 4 months, about 2 weeks to 3 months, or about 1 month to 3 months.

For temporal monitoring, there can be more than 2 time points of measurement, *e.g.,* at least 3 time points, at least 4 time points, or at least 5 time points.

The terminology used herein was chosen to best explain the principles of the method, the practical application or technical improvement over technologies found in the marketplace, or to enable others of ordinary skill in the art to understand the methods disclosed here.

All definitions, as defined and used herein, should be understood to control over dictionary definitions, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, *i.e.,* elements that are conjunctively present in some cases and disjunctively present in other cases. Multiple elements listed with "and/or" should be construed in the same fashion, *i.e.,* "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one example, to A only (optionally including elements other than B); in another example, to B only (optionally including elements other than A); in yet another example, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, *i.e*., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (*i.e*. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one example, to at least one, optionally including more than one, A, with no B present (and optionally including elements other than B); in another example, to at least one, optionally including more than one, B, with no A present (and optionally including elements other than A); in yet another example, to at least one, optionally including more than one, A, and at least one, optionally including more than one, B (and optionally including other elements); etc.

As used herein, the term "about" means a range of values that are similar to the stated reference value. In certain instances, the term "about" refers to a range of values that fall within 10 percent or less (*e.g.,* 10%, 9%, 8%, 7%, 6%, 5%, 4%, 3%, 2%, or 1%) of the stated reference value.

As used herein, the term "significantly" means at least 5%, at least 10%, at least 15%, at least 20%, at least 25%, at least 30%, at least 35%, at least 40%, at least 45%, or at least 50%.

As used herein, the term "renal clearable," when applied to nanoparticles or nanomaterials, means that the nanoparticles or nanomaterials have a 1-hour or a 2-hour renal clearance efficiency of at least 5%, at least 10%, at least 15%, at least 20%, at least 25%, at least 30%, at least 35%, at least 40%, at least 45%, or at least 50% of the injected dose. The renal clearable nanoparticles or nanomaterials may have a 1-hour renal clearance efficiency of at least 5%, at least 10%, at least 15%, at least 20%, at least 25%, at least 30%, at least 35%, at least 40%, at least 45%, or at least 50% of the injected dose. The renal clearable nanoparticles or nanomaterials may have a 1-hour renal clearance efficiency in the range of about 5% to 100% of injected dose. Optionally, the renal clearable nanoparticles or nanomaterials have a 1-hour or a 2-hour renal clearance efficiency of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% of the injected dose. The renal clearable nanoparticles or nanomaterials may have a 1-hour renal clearance efficiency of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% of the injected dose.

As used herein, the term "clearance efficiency" means the amount of nanoparticles excreted into urine at a certain time point post administration divided by the amount of nanoparticles administered to the subject. As such, clearance efficiency can be time dependent.

As used herein, the term "kidney function" means generally the functional status of the kidney. The term "kidney function" can be used to describe the function of a healthy kidney (healthy kidney function) or the function of a kidney that is impaired or injured or a kidney having a disease or disorder (impaired kidney function). Kidney function may be represented by the excretory capacity of the kidney.

As used herein, the term "kidney dysfunction" means that the function of the kidney is below the function of a healthy kidney, including about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, or about 95% of the function of a healthy kidney, *e.g.,* the kidney when the subject is healthy, or of the average kidney function of a group of healthy subjects.

As used herein, the term "early stage," when applied to kidney dysfunction, injury, or disease, means a stage when the kidney dysfunction, injury, or disease cannot be identified by current kidney injury/function biomarkers, such as serum biomarkers (*e.g*., blood urea nitrogen or serum creatinine) and urine biomarkers (*e.g*., urine protein, urine protein-to-urine creatinine ratio, or urine albumin-to-urine creatinine ratio).

As used herein, the term "late stage," when applied to kidney dysfunction, injury, or disease, means a stage when the kidney dysfunction, injury, or disease can be identified by current kidney injury/function biomarkers, such as serum biomarkers (*e.g.*, blood urea nitrogen or serum creatinine) and urine biomarkers (*e.g*., urine protein, urine protein-to-urine creatinine ratio, or urine albumin-to-urine creatinine ratio).

As used herein, the term "dose" means the amount of nanoparticles that are administered to a subject. A dose can have a variety of units, such as mg/kg (milligram of nanoparticles per kilogram of body weight) and mmol/kg (millimole of nanoparticles per kilogram of body weight).

As used herein, the term "subject" means a human or other vertebrate animal. The subject may be a human having kidney dysfunction, injury, or disease. The subject may be a human susceptible to having kidney dysfunction, injury, or disease. The subject may be a human suffering from a disease or disorder that is prone to cause kidney dysfunction, injury, or disease, such as an autoimmune disease or disorder (*e.g*., rheumatoid arthritis, lupus, Inflammatory bowel disease, multiple sclerosis, Type 1 diabetes mellitus, Guillain-Barre syndrome, chronic inflammatory demyelinating polyneuropathy, psoriasis, Graves' disease, Hashimoto's thyroiditis, myasthenia gravis, or vasculitis). The subject may be a human receiving a treatment for a disease or disorder, including chemotherapy or an immune-oncological treatment. Examples of chemotherapy drugs include, but are not limited to, alkylating agents, nitrosoureas, anti-metabolites, plant alkaloids, anti-tumor antibiotics, hormonal agents, and biological response modifiers. The subject may be a human receiving an immuno-oncological treatment.

In the claims, as well as in the specification above, all transitional phrases such as "comprising," "including," "carrying," "having," "containing," "involving," "holding," "composed of," and the like are to be understood to be open-ended, *i.e.,* to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

### EXAMPLES

### Example 1.

Gold nanomaterials have been extensively explored in disease diagnosis and treatment *in vivo.* They can serve as contrast imaging agents for X-ray imaging or deliver other imaging agents to the diseased organs and tissues. Due to large size or serum protein adsorption, conventional gold nanomaterials are often accumulated in the reticuloendothelial system (RES) organs (*e.g*., liver and spleen) for a long time after injection. For example, liver uptake of gold nanomaterials was about 15-90 % ID at 24 h post injection. Gold nanoparticles are often considered biocompatible because they are relatively inert in biological environment. Although the FDA has not approved any gold-based nanomedicines, many types of gold nanoparticles are currently under preclinical development and two types of gold nanoparticles, CYT-6091 and AuroShell, have been tested in clinical trials for cancer treatment.

To further reduce the potential toxicity of gold nanoparticles related to their long-term body accumulation, a series of renal clearable gold nanoparticles were developed and their applications in disease diagnosis was conducted in the past decade. For example, renal clearable glutathione-coated gold nanoparticles (GS-AuNPs) were synthesized that show a maximum emission at 810 nm and have an average core size of 2.5 nm and hydrodynamic diameter of 3.3 nm. Each 2.5 nm GS-AuNP contains 640 gold atoms. Different from conventional gold nanomaterials, these GS-AuNPs were dominantly eliminated from the body by the kidneys. At 24 h post-intravenous injection, about 50 % ID were excreted in the urine of mice or non-human primates. The GS-AuNPs were also highly biocompatible: the no-observed-adverse-effect-level (NOAEL) of these nanoparticles was measured to be > 1000 mg/kg in CD-1 mice and >250 mg/kg in cynomolgus monkeys.

To test whether GS-AuNPs can serve as an exogenous marker for kidney function evaluation, the cisplatin-induced acute kidney injury model, which is one of the most widely used models for understanding of drug-induced acute kidney injury, was used. Briefly, 16 male CD-1 mice (30-35 g, 8-10 week-old) were randomly divided into two groups: (1) twelve mice received a single intraperitoneal injection of cisplatin at 15 mg/kg on Day 0, (2) four mice received a single intraperitoneal injection of phosphate-buffered saline (PBS, control group) on Day 0. Four mice receiving cisplatin were randomly selected to measure the renal clearance and plasma clearance of GS-AuNPs on Day 1, 2, 3, respectively. The control study using 4 mice receiving PBS was performed on Day 3. 183 mg/kg GS-AuNPs was intravenously injected into the mice. At 1-h post intravenous injection of GS-AuNPs, urine, blood, and kidneys were collected to measure the 1-h renal clearance, blood concentration and kidney accumulation of AuNPs with inductively coupled plasma mass spectrometry (ICP-MS). In addition, serum samples were also collected for the measurement of BUN and serum creatinine. Kidneys were processed for pathological analysis to identify structural changes of tissues. Since tubular cell apoptosis plays an essential pathogenic role for cisplatin-induced acute kidney injury, cell apoptosis was also examined by using terminal deoxynucleotidyl transferase-mediated digoxigenin-deoxyuridine nick-end labeling (TUNEL) staining.

Using TUNEL assay, two different stages of the cisplatin-induced acute kidney injury were identified. Stage I was an early stage where apoptosis was identified in the kidney tissues of cisplatin-injected mice, in contrast to the negligible apoptosis in the normal kidneys of control group **(****FIG. 1****);** however, BUN and serum creatinine levels of these mice remained in the normal range obtained from control group (**FIG. 2** and **FIG. 3**). Stage II was a mild stage that showed more pronounced apoptosis in the kidney when compared to Stage I (**FIG. 1**) and also exhibited a significant increase in the levels of BUN and serum creatinine than normal control (**FIG. 2** and **FIG. 3**).

In contrast to the silence of BUN and serum creatinine at Stage I, an early stage, clearance of renal clearable GS-AuNPs was highly sensitive to the functional status of the kidney in cisplatin-induced acute kidney injury model. Compared with control group, the urine excretion of GS-AuNPs decreased and blood retention was prolonged in the cisplatin model. The 1-h urine excretion of GS-AuNPs was well correlated with the degrees of apoptosis of kidney that were assessed by TUNEL assay. Using ICP-MS, we measured the amount of gold in the urine. The 1-h renal clearance efficiency decreased from 48.5 ± 6.8 % ID in control mice with normal kidney function to 27.2 ± 4.5 % ID in mice with kidney dysfunction at an early stage when BUN and serum creatinine were silent (Stage I), and 11.9 ± 4.9 % ID in mice with mild kidney dysfunction (Stage II) **(****FIG. 4****).** Consistent with the urine test results, blood test showed that the blood concentration of GS-AuNPs was well correlated with the stages of cisplatin-induced injury. At 1 h post intravenous injection, the amount of gold in blood increased from 0.8 ± 0.4 % ID/g in control mice with normal kidney function to 3.7 ± 1.6 % ID/g in mice with kidney dysfunction at an early stage when BUN and serum creatinine were silent (Stage I), and 3.4 ± 0.5 % ID/g in mice with mild kidney dysfunction (Stage II) **(****FIG. 5****).** Moreover, a gradual increase in the kidney accumulation of GS-AuNPs was found at 1 h post injection as the kidney injury became more severely (**FIG. 6**)**.** At 1 h post intravenous injection, the amounts of gold in the kidney were 5.6 ± 1.3, 21.5 ± 4.6, and 29.8 ± 2.6 % ID per gram of tissue (% ID/g) for control group, cisplatin model at Stage I, and cisplatin model at Stage II, respectively.

Moreover, a difference was also observed between the control group and cisplatin model in the emission spectrum of GS-AuNPs excreted into urine. The renal clearable near-infrared-emitting GS-AuNPs showed a maximum emission at 810 nm and had very weak emission at 627 nm before they were introduced into the body ("Pre-injection", **FIG. 7**)**.** After being intravenously injected to the control mice, circulating in the body, and being excreted into urine, GS-AuNPs exhibited a 6.8-fold increase of the emission intensity at 627 nm in comparison with "Pre-injection". This dramatic increase of 627 nm suggested an enhanced surface coverage of the thiol ligands on the AuNPs, according to our previous understanding of the size-independent emission of these GS-AuNPs. The reason could be due to binding of biological thiols to the gold surface during the circulation in the body. Regarding the cisplatin model, GS-AuNPs excreted into urine only showed a 2.4-fold increase of the 627 nm emission compared with "Pre-injection", suggesting that the level of biological thiols was significantly reduced in the cisplatin model.

### Example 2.

To test whether the renal clearance and plasma clearance of GS-AuNPs can be used for early diagnosis and monitoring of kidney dysfunction caused by lupus, a robust genetic mouse model of lupus - MRL/MpJ-*Fas^{lpr}*/J **(MRL-*lpr*)** was chosen, and MRL/MpJ mice was used as a control. The mice were purchased from The Jackson Laboratory. The MRL-*lpr* lupus mouse model recapitulates many clinical features of human lupus, including kidney damage, and has been used extensively in preclinical lupus research. In this genetic model of lupus, as the mice age the kidney damage increases. The urine protein level of an MRL-*lpr* mouse was increased by 10-times at age of 10 weeks (283 mg/dL) compared to a normal value of urine protein (10-30 mg/dL), and was further increased by 100-times at ages of 12-14 weeks (2200-2400 mg/dL), indicating the progression of lupus nephritis **(****FIG. 8****).** Consistent with the increase of urine protein level, this MRL-*lpr* mouse had a gradual decrease in 1-hour renal clearance of GS-AuNPs (5 mg/kg) after intravenous injection from a normal value of 53.8±1.2 percent of injected dose (%ID, n=4) to 45.7, 31.8, and 9.4 %ID at age of 10, 12, and 14 weeks, corresponding to decreases of 15.0, 40.9, and 82.5% in 1-hour renal clearance compared to normal value **(****FIG. 9****).** These results indicated the renal clearance of GS-AuNPs decreased with the progression of lupus nephritis, implying that the gold amount in the urine can be used to detect the kidney damages caused by lupus. Interestingly, in three MRL-*lpr* lupus mice having normal urine protein levels at age of 10, 12, and 14 weeks **(****FIG. 10****),** a dramatic decrease in the 1-hour renal clearance of GS-AuNPs was observed from 56.4±7.5 to 42.7±14.9 and 26.4±6.3 %ID with time during this period of time **(****FIG. 11****).**

When the mice were at the age of 8 weeks, the feasibility of distinguishing the lupus model from control group using blood clearance of GS-AuNPs was also tested. On Day 0, urine samples were collected for measurement of urine protein (proteinuria). Serum samples were also collected for creatinine measurement. On Day 1, after intravenous injection of 5 mg/kg GS-AuNPs into the mice, the blood for each mouse was collected at 5 min and 1 h, and then used ICP-MS to measure gold in the urine. **FIG. 12** shows that all mice (including MRL-*lpr* lupus model and control MRL/MpJ mice) were silent in proteinuria. However, the blood retention of GS-AuNPs was prolonged in lupus model compared to that in control MRL/MpJ group **(****FIG. 13****).** Between the MRL-*lpr* lupus model and control MRL/MpJ mice, a significant difference was found in the decrease percentage of blood concentration of renal clearable GS-AuNPs at 1 h compared to that at 5 min (=[(blood concentration at 5 min- blood concentration at 1 h)/ blood concentration at 5 min] x 100%).

### Example 3.

If the glomerulus is injured, higher 1-hour renal clearance of GS-AuNPs (60-65 % ID than a normal value of 53.8±1.2 % ID was observed **(****FIG. 15****)** even though the urine protein levels of the diseased mice and control ones were all below 60 mg/dL and remaining in the normal range **(****FIG. 16****).**

## Claims

1. A method of evaluating kidney function of a subject, the method comprising:
(a) characterizing nanoparticles in a urine sample collected from a subject with a measurement process to obtain a characteristic parameter; and
(b) comparing the characteristic parameter with a reference value, thereby evaluating the kidney function,
wherein a first plurality of nanoparticles having a first dose has been administered to the subject and the urine sample has been collected within a first period of time after the administration.

2. The method of claim 1, wherein the first period of time is about 30 minutes to 24 hours.

3. A method of evaluating kidney function of a subject, the method comprising:
(a) characterizing nanoparticles in a blood sample collected from a subject, with a measurement process to obtain a characteristic parameter; and
(b) comparing the characteristic parameter with a reference value, thereby evaluating the kidney function,
wherein a first plurality of nanoparticles having a first dose has been administered to the subject and the blood sample has been collected within a first period of time after the administration.

4. The method of claim 3, wherein the first period of time is about 5 mins to 1 hour.

5. The method of any one of claims 1-4, wherein the reference value is a control characteristic parameter measured for a control group having normal kidney function.

6. The method of any one of claims 1-5, further comprising indicating kidney dysfunction or injury when the characteristic parameter is significantly different from the control characteristic parameter or reference value.

7. The method of claim 6, wherein the kidney dysfunction is caused by drug-induced nephrotoxicity, an autoimmune disease, kidney failure, chronic kidney disease, cystic kidney disease, kidney inflammatory disease, kidney fibrosis, autosomal dominant polycystic kidney disease, an immuno-oncological treatment, an infection or a combination thereof, preferably wherein the autoimmune disease is lupus or wherein the kidney inflammatory disease is lupus nephritis.

8. A method of monitoring kidney function of a subject, the method comprising:
(a) characterizing nanoparticles in a first urine sample with a measurement process to obtain a first characteristic parameter;
(b) characterizing nanoparticles in a second urine sample with the measurement process to obtain a second characteristic parameter; and
(c) comparing the first characteristic parameter with the second characteristic parameter, thereby monitoring the kidney function over time;
wherein a first plurality of nanoparticles having a first dose has been administered to the subject and the first urine sample has been collected within a first period of time after the administration of the first plurality of nanoparticles; and
wherein a second plurality of nanoparticles having the first dose has been administered to the subject after a second period of time and the second urine sample has been collected within a third period of time after the administration of the second plurality of nanoparticles.

9. A method of monitoring kidney function of a subject, the method comprising:
(a) characterizing a first plurality of nanoparticles in a first blood sample collected within a first period of time from the subject administered with the first plurality of nanoparticles, with a measurement process to obtain a first characteristic parameter;
(b) after a second period of time after step (c), characterizing a second plurality of nanoparticles in a second blood sample collected within a third period of time from the subject administered with the second plurality of nanoparticles, with the measurement process to obtain a second characteristic parameter; and
(c) comparing the first characteristic parameter with the second characteristic parameter, thereby monitoring the kidney function over time.

10. The method of claim 8 or 9, wherein the second period of time is about 1 hour to 30 days for acute kidney disease, or wherein the second period of time is about two weeks to 12 months for chronic kidney disease.

11. The method of any one of claims 1-9, wherein the nanoparticles are renal clearable.

12. The method of claim 11, wherein the nanoparticles have a 1-hour or 2-hour renal clearance efficiency in the range of 5 to 100 percent of injected dose (% ID); or wherein the nanoparticles comprise gold, silver, copper, platinum, palladium, silica, carbon, silicon, iron oxide, FeS, CdSe, CdS, CuS, an organic material, or a combination thereof.

13. The method of any one of claims 1-12, wherein the nanoparticles are coated with a ligand selected from the group consisting of glutathione, thiol-functionalized polyethylene glycol, cysteamine, cysteine, homocysteine, a dipeptide containing cysteine, a dipeptide containing homocysteine, a peptide having more than three amino acids, and a combination thereof.

14. The method of claim 13, wherein the dipeptide containing cysteine includes cysteine-glycine or cysteine-glutamic acid; or wherein the dipeptide containing homocysteine includes homocysteine-glycine or homocysteine-glutamic acid; or wherein the ligand is conjugated with a fluorescent dye; or wherein the ligand is glutathione.

15. The method of any one of claims 1-14, wherein the nanoparticles fluoresce in a range of 500 to 850 nm; or wherein the nanoparticles fluoresce in a range of 1000 to 1700 nm.

16. The method of claim 8, wherein the first period of time is about 30 minutes to 24 hours for the urine sample; or wherein the third period of time is about 30 minutes to 24 hours for the urine sample.

17. The method of claim 9, wherein the first period of time is about 5 mins to 1 hour for the blood sample; or wherein the third period of time is about 5 mins to 24 hours for the blood sample.

18. The method of any one of claims 1-17, wherein the measurement process includes one or more processes selected from the group consisting of: measuring a concentration of the nanoparticles, measuring an amount of the nanoparticles, measuring clearance efficiency, analyzing a composition of ligands on the nanoparticle surface, measuring size distribution of the nanoparticles, measuring an absorption spectrum of the nanoparticles, measuring an emission spectrum of the nanoparticles, measuring an excitation spectrum of the nanoparticles, measuring a photoacoustic signal of the nanoparticles, measuring radioactivity of the nanoparticles, or a combination thereof, preferably wherein the measurement process includes inductively coupled plasma mass spectrometry (ICP-MS) or inductively coupled plasma optical emission spectroscopy (ICP-OES).

19. The method of any one of claims 1-18, wherein the characteristic parameter is selected from the group consisting of: a concentration of the nanoparticles, an amount of the nanoparticles, clearance efficiency, a composition of ligands on the nanoparticle surface, one type of surface ligand, size distribution of the nanoparticles, surface charge of the nanoparticles, an absorption spectrum of the nanoparticles, an emission spectrum of the nanoparticles, an excitation spectrum of the nanoparticles, a photoacoustic signal of the nanoparticles, radioactivity of the nanoparticles, and a combination thereof.

20. The method of claim 19, wherein:
(ai) the characteristic parameter is the concentration of the nanoparticles in the blood sample of the subject;
(aii) the control characteristic parameter is the control concentration of the nanoparticles in the blood sample of the control group; and
(aii) if the concentration is significantly different from the control concentration, then it indicates kidney dysfunction or injury; or
(bi) the characteristic parameter is the clearance efficiency of the nanoparticles in the subject;
(bii) the control characteristic parameter is the control clearance efficiency of the nanoparticles in the control group; and
(biii) if the clearance efficiency is significantly different from the control clearance efficiency, then it indicates kidney dysfunction or injury; or
(ci) the characteristic parameter is the emission spectrum of the nanoparticles in the urine sample of the subject;
(cii) the control characteristic parameter is the control emission spectrum of the nanoparticles in the urine sample of the control group; and
(ciii) if the emission spectrum is significantly different from the control emission spectrum, then it indicates kidney dysfunction or injury.

21. The method of any one of claims 1-20, wherein the administration is to be intravenous, intraperitoneal, subcutaneous, or intraarterial.

## Patentansprüche

1. Verfahren zur Beurteilung einer Nierenfunktion eines Probanden, wobei das Verfahren umfasst:
(a) Charakterisieren von Nanopartikeln in einer von einem Probanden entnommenen Urinprobe mit einem Messverfahren, um einen charakteristischen Parameter zu erhalten; und
(b) Vergleichen des charakteristischen Parameters mit einem Referenzwert, wodurch die Nierenfunktion beurteilt wird,
wobei dem Probanden eine erste Vielzahl von Nanopartikeln mit einer ersten Dosis verabreicht wurde und die Urinprobe innerhalb eines ersten Zeitraums nach der Verabreichung entnommen wurde.

2. Verfahren nach Anspruch 1, wobei der erste Zeitraum etwa 30 Minuten bis 24 Stunden beträgt.

3. Verfahren zur Beurteilung einer Nierenfunktion eines Probanden, wobei das Verfahren umfasst:
(a) Charakterisieren von Nanopartikeln in einer von einem Probanden entnommenen Blutprobe mit einem Messverfahren, um einen charakteristischen Parameter zu erhalten; und
(b) Vergleichen des charakteristischen Parameters mit einem Referenzwert, wodurch die Nierenfunktion beurteilt wird,
wobei dem Probanden eine erste Vielzahl von Nanopartikeln mit einer ersten Dosis verabreicht wurde und die Blutprobe innerhalb eines ersten Zeitraums nach der Verabreichung entnommen wurde.

4. Verfahren nach Anspruch 3, wobei der erste Zeitraum etwa 5 Minuten bis 1 Stunde beträgt.

5. Verfahren nach einem der Ansprüche 1-4, wobei der Referenzwert ein charakteristischer Kontrollparameter ist, der für eine Kontrollgruppe mit einer normalen Nierenfunktion gemessen wird.

6. Verfahren nach einem der Ansprüche 1-5, das ferner das Anzeigen einer Nierendysfunktion oder Nierenverletzung umfasst, wenn sich der charakteristische Parameter signifikant von dem charakteristischen Kontrollparameter oder dem Referenzwert unterscheidet.

7. Verfahren nach Anspruch 6, wobei die Nierendysfunktion verursacht wird durch medikamenteninduzierte Nephrotoxizität, eine Autoimmunerkrankung, Nierenversagen, eine chronische Nierenerkrankung, eine zystische Nierenerkrankung, eine entzündliche Nierenerkrankung, Nierenfibrose, eine autosomal dominante polyzystische Nierenerkrankung, eine immunonkologische Therapie, eine Infektion oder eine Kombination davon, wobei die Autoimmunerkrankung vorzugsweise Lupus ist, oder wobei die entzündliche Nierenerkrankung Lupus-Nephritis ist.

8. Verfahren zur Beurteilung einer Nierenfunktion eines Probanden, wobei das Verfahren umfasst:
(a) Charakterisieren von Nanopartikeln in einer ersten Urinprobe mit einem Messverfahren, um einen ersten charakteristischen Parameter zu erhalten;
(b) Charakterisieren von Nanopartikeln in einer zweiten Urinprobe mit dem Messverfahren, um einen zweiten charakteristischen Parameter zu erhalten; und
(c) Vergleichen des ersten charakteristischen Parameters mit dem zweiten charakteristischen Parameter, wodurch die Nierenfunktion im Laufe der Zeit überwacht wird;
wobei dem Probanden eine erste Vielzahl von Nanopartikeln mit einer ersten Dosis verabreicht wurde und die erste Urinprobe innerhalb eines ersten Zeitraums nach der Verabreichung der ersten Vielzahl von Nanopartikeln entnommen wurde; und
wobei dem Probanden eine zweite Vielzahl von Nanopartikeln mit der ersten Dosis nach einem zweiten Zeitraum verabreicht wurde und die zweite Urinprobe innerhalb eines dritten Zeitraums nach der Verabreichung der zweiten Vielzahl von Nanopartikeln entnommen wurde.

9. Verfahren zur Beurteilung einer Nierenfunktion eines Probanden, wobei das Verfahren umfasst:
(a) Charakterisieren einer ersten Vielzahl von Nanopartikeln in einer ersten Blutprobe, die innerhalb eines ersten Zeitraums von dem Probanden entnommen wird, dem die erste Vielzahl von Nanopartikeln verabreicht wurde, mit einem Messverfahren, um einen ersten charakteristischen Parameter zu erhalten;
(b) nach einem zweiten Zeitraum nach Schritt (c), Charakterisieren einer zweiten Vielzahl von Nanopartikeln in einer zweiten Blutprobe, die innerhalb eines dritten Zeitraums von dem Probanden entnommen wird, dem die zweite Vielzahl von Nanopartikeln verabreicht wurde, mit dem Messverfahren, um einen zweiten charakteristischen Parameter zu erhalten; und
(c) Vergleichen des ersten charakteristischen Parameters mit dem zweiten charakteristischen Parameter, wodurch die Nierenfunktion im Laufe der Zeit überwacht wird.

10. Verfahren nach Anspruch 8 oder 9, wobei der zweite Zeitraum etwa 1 Stunde bis 30 Tage für eine akute Nierenerkrankung beträgt, oder wobei der zweite Zeitraum etwas zwölf Wochen bis 12 Monate für eine chronische Nierenerkrankung beträgt.

11. Verfahren nach einem der Ansprüche 1-9, wobei die Nanopartikel renal klärbar sind.

12. Verfahren nach Anspruch 11, wobei die Nanopartikel eine 1-stündige oder 2-stündige renale Clearance-Effizienz im Bereich von 5 bis 100 Prozent der injizierten Dosis (% ID) aufweisen; oder wobei die Nanopartikel Gold, Silber, Kupfer, Platin, Palladium, Siliciumdioxid, Kohlenstoff, Silizium, Eisenoxid, FeS, CdSe, CdS, CuS, ein organisches Material, oder eine Kombination davon enthalten.

13. Verfahren nach einem der Ansprüche 1-12, wobei die Nanopartikel mit einem Ligand beschichtet sind, der ausgewählt ist aus der Gruppe bestehend aus Glutathion, Thiol-funktionalisiertem Polyethylenglykol, Cysteamin, Cystein, Homocystein, einem cysteinhaltigem Dipeptid, einem homocysteinhaltigen Dipeptid, einem Peptid mit mehr als drei Aminosäuren, und einer Kombination davon.

14. Verfahren nach Anspruch 13, wobei das cysteinhaltige Dipeptid ein Cystein-Glycin oder Cystein-Glutaminsäure enthält; oder wobei das homocysteinhaltige Dipeptid ein Homocystein-Glycin oder Homocystein-Glutaminsäure enthält; oder wobei der Ligand mit einer Fluoreszenzfarbe konjugiert ist; oder wobei der Ligand Glutathion ist.

15. Verfahren nach einem der Ansprüche 1-14, wobei die Nanopartikel in einem Bereich von 500 bis 850 nm fluoreszieren; oder wobei die die Nanopartikel in einem Bereich von 1000 bis 1700 nm fluoreszieren.

16. Verfahren nach Anspruch 8, wobei der erste Zeitraum etwa 30 Minuten bis 24 Stunden für die Urinprobe beträgt; oder wobei der dritte Zeitraum etwa 30 Minuten bis 24 Stunden für die Urinprobe beträgt.

17. Verfahren nach Anspruch 9, wobei der erste Zeitraum etwa 5 Minuten bis 1 Stunde für die Blutprobe beträgt; oder wobei der dritte Zeitraum etwa 5 Minuten bis 24 Stunden für die Blutprobe beträgt.

18. Verfahren nach einem der Ansprüche 1-17, wobei das Messverfahren ein oder mehrere Verfahren umfasst, die ausgewählt sind aus der Gruppe bestehend aus: Messen einer Konzentration der Nanopartikel, Messen einer Menge an Nanopartikeln, Messen einer Clearance-Effizienz, Analysieren einer Zusammensetzung von Liganden auf der Nanopartikeloberfläche, Messen einer Größenverteilung der Nanopartikel, Messen eines Absorptionsspektrums der Nanopartikel, Messen eines Emissionsspektrums der Nanopartikel, Messen eines Anregungsspektrums der Nanopartikel, Messen eines photoakustischen Signals der Nanopartikel, Messen einer Radioaktivität der Nanopartikel, oder eine Kombination davon, wobei das Messverfahren vorzugsweise Massenspektrometrie mit induktiv gekoppeltem Plasma (ICP-MS) oder optische Emissionsspektrometrie mit induktiv gekoppeltem Plasma (ICP-OES) umfasst.

19. Verfahren nach einem der Ansprüche 1-18, wobei der charakteristische Parameter ausgewählt ist aus der Gruppe bestehend aus: einer Konzentration der Nanopartikel, einer Menge von Nanopartikeln, einer Clearance-Effizienz, einer Zusammensetzung von Liganden auf der Nanopartikeloberfläche, einer Art von Oberflächenliganden, einer Größenverteilung der Nanopartikel, einer Oberflächenveränderung der Nanopartikel, einem Absorptionsspektrum der Nanopartikel, einem Emissionsspektrum der Nanopartikel, einem Anregungsspektrum der Nanopartikel, einem photoakustischen Signal der Nanopartikel, einer Radioaktivität der Nanopartikel, oder einer Kombination davon.

20. Verfahren nach Anspruch 19, wobei:
(ai) der charakteristische Parameter die Konzentration der Nanopartikel in der Blutprobe des Probanden ist;
(aii) der charakteristische Kontrollparameter die Kontrollkonzentration der Nanopartikel in der Blutprobe der Kontrollgruppe ist; und
(aii) wenn sich die Konzentration signifikant von der Kontrollkonzentration unterscheidet, es dann eine Nierendysfunktion oder Nierenverletzung anzeigt; oder
(bi) der charakteristische Parameter die Clearance-Effizienz der Nanopartikel in dem Probanden ist;
(bii) der charakteristische Kontrollparameter die Kontroll-Clearance-Effizienz der Nanopartikel in der Kontrollgruppe ist; und
(biii) wenn sich die Clearance-Effizienz signifikant von der Kontroll-Clearance-Effizienz unterscheidet, es dann eine Nierendysfunktion oder Nierenverletzung anzeigt; oder
(ci) der charakteristische Parameter das Emissionsspektrum der Nanopartikel in der Urinprobe des Probanden ist;
(cii) der charakteristische Kontrollparameter das Kontroll-Emissionsspektrum der Nanopartikel in der Urinprobe der Kontrollgruppe ist; und
(ciii) wenn sich das Emissionsspektrum signifikant von dem Kontroll-Emissionsspektrum unterscheidet, es dann eine Nierendysfunktion oder Nierenverletzung anzeigt.

21. Verfahren nach einem der Ansprüche 1-20, wobei die Verabreichung intravenös, intraperitoneal, subkutan oder intraarteriell erfolgen soll.

## Revendications

1. Méthode d'évaluation de la fonction rénale d'un sujet, la méthode comprenant :
(a) la caractérisation de nanoparticules dans un échantillon urinaire prélevé chez un sujet avec un procédé de mesure pour obtenir un paramètre caractéristique ; et
(b) la comparaison du paramètre caractéristique avec une valeur de référence, permettant ainsi d'évaluer la fonction rénale,
dans laquelle une première pluralité de nanoparticules présentant une première dose ont été administrées au sujet et l'échantillon urinaire a été prélevé au cours d'une première période après l'administration.

2. Méthode selon la revendication 1, dans laquelle la première période est d'environ 30 minutes à 24 heures.

3. Méthode d'évaluation de la fonction rénale d'un sujet, la méthode comprenant :
(a) la caractérisation de nanoparticules dans un échantillon sanguin prélevé chez un sujet, avec un procédé de mesure pour obtenir un paramètre caractéristique ; et
(b) la comparaison du paramètre caractéristique avec une valeur de référence, permettant ainsi d'évaluer la fonction rénale,
dans laquelle une première pluralité de nanoparticules présentant une première dose ont été administrées au sujet et l'échantillon sanguin a été prélevé au cours d'une première période après l'administration.

4. Méthode selon la revendication 3, dans laquelle la première période est d'environ 5 minutes à 1 heure.

5. Méthode selon l'une quelconque des revendications 1-4, dans laquelle la valeur de référence est un paramètre caractéristique témoin mesuré pour un groupe témoin présentant une fonction rénale normale.

6. Méthode selon l'une quelconque des revendications 1-5, comprenant en outre l'indication d'un dysfonctionnement ou d'une lésion rénal(e) lorsque le paramètre caractéristique est considérablement différent du paramètre caractéristique témoin ou de la valeur de référence.

7. Méthode selon la revendication 6, dans laquelle le dysfonctionnement rénal est causé par une néphrotoxicité médicamenteuse, une maladie auto-immune, une insuffisance rénale, une maladie rénale chronique, une maladie kystique rénale, une maladie inflammatoire du rein, une fibrose rénale, une maladie polykystique rénale autosomique dominante, un traitement immuno-oncologique, une infection ou une combinaison de ceux-ci, de préférence dans laquelle la maladie auto-immune est un lupus ou dans laquelle la maladie inflammatoire du rein est une néphrite lupique.

8. Méthode de surveillance de la fonction rénale d'un sujet, la méthode comprenant :
(a) la caractérisation de nanoparticules dans un premier échantillon urinaire avec un procédé de mesure pour obtenir un premier paramètre caractéristique ;
(b) la caractérisation de nanoparticules dans un second échantillon urinaire avec le procédé de mesure pour obtenir un second paramètre caractéristique ; et
(c) la comparaison du premier paramètre caractéristique avec le second paramètre caractéristique, permettant ainsi de surveiller la fonction rénale au fil du temps ;
dans laquelle une première pluralité de nanoparticules présentant une première dose ont été administrées au sujet et le premier échantillon urinaire a été prélevé au cours d'une première période après l'administration de la première pluralité de nanoparticules ; et
dans laquelle une seconde pluralité de nanoparticules présentant la première dose ont été administrées au sujet après une deuxième période et le second échantillon urinaire a été prélevé au cours d'une troisième période après l'administration de la seconde pluralité de nanoparticules.

9. Méthode de surveillance de la fonction rénale d'un sujet, la méthode comprenant :
(a) la caractérisation d'une première pluralité de nanoparticules dans un premier échantillon sanguin prélevé au cours d'une première période chez le sujet auquel ont été administrées la première pluralité de nanoparticules, avec un procédé de mesure pour obtenir un premier paramètre caractéristique ;
(b) après une deuxième période suite à l'étape (c), la caractérisation d'une seconde pluralité de nanoparticules dans un second échantillon sanguin prélevé au cours d'une troisième période chez le sujet auquel ont été administrées la seconde pluralité de nanoparticules, avec le procédé de mesure pour obtenir un second paramètre caractéristique ; et
(c) la comparaison du premier paramètre caractéristique avec le second paramètre caractéristique, permettant ainsi de surveiller la fonction rénale au fil du temps.

10. Méthode selon la revendication 8 ou 9, dans laquelle la deuxième période est d'environ 1 heure à 30 jours pour une maladie rénale aiguë, ou dans laquelle la deuxième période est d'environ deux semaines à 12 mois pour une maladie rénale chronique.

11. Méthode selon l'une quelconque des revendications 1-9, dans laquelle les nanoparticules sont éliminées par clairance rénale.

12. Méthode selon la revendication 11, dans laquelle les nanoparticules présentent une efficacité d'élimination par clairance rénale de 1 heure ou 2 heures dans la plage de 5 à 100 pour cent de dose injectée (% ID) ; ou dans laquelle les nanoparticules comprennent de l'or, de l'argent, du cuivre, du platine, du palladium, de la silice, du carbone, du silicium, de l'oxyde de fer, du FeS, du CdSe, du CdS, du CuS, un matériau organique, ou une combinaison de ceux-ci.

13. Méthode selon l'une quelconque des revendications 1-12, dans laquelle les nanoparticules sont enrobées d'un ligand sélectionné dans le groupe consistant en du glutathion, du polyéthylène glycol à fonction thiol, de la cystéamine, de la cystéine, de l'homocystéine, un dipeptide contenant de la cystéine, un dipeptide contenant de l'homocystéine, un peptide présentant plus de trois acides aminés, et une combinaison de ceux-ci.

14. Méthode selon la revendication 13, dans laquelle le dipeptide contenant de la cystéine inclut un acide cystéine-glycine ou cystéine-glutamique ; ou dans laquelle le dipeptide contenant de l'homocystéine inclut un acide homocystéine-glycine ou homocystéine-glutamique ; ou dans laquelle le ligand est conjugué avec un colorant fluorescent ; ou dans laquelle le ligand est le glutathion.

15. Méthode selon l'une quelconque des revendications 1-14, dans laquelle les nanoparticules émettent une fluorescence dans une plage de 500 à 850 nm ; ou dans laquelle les nanoparticules émettent une fluorescence dans une plage de 1000 à 1700 nm.

16. Méthode selon la revendication 8, dans laquelle la première période est d'environ 30 minutes à 24 heures pour l'échantillon urinaire ; ou dans laquelle la troisième période est d'environ 30 minutes à 24 heures pour l'échantillon urinaire.

17. Méthode selon la revendication 9, dans laquelle la première période est d'environ 5 minutes à 1 heure pour l'échantillon sanguin ; ou dans laquelle la troisième période est d'environ 5 minutes à 24 heures pour l'échantillon sanguin.

18. Méthode selon l'une quelconque des revendications 1-17, dans laquelle le procédé de mesure inclut un ou plusieurs procédés sélectionnés dans le groupe consistant en : la mesure d'une concentration des nanoparticules, la mesure d'une quantité des nanoparticules, la mesure d'une efficacité d'élimination par clairance, l'analyse d'une composition de ligands sur la surface de nanoparticule, la mesure d'une distribution granulométrique des nanoparticules, la mesure d'un spectre d'absorption des nanoparticules, la mesure d'un spectre d'émission des nanoparticules, la mesure d'un spectre d'excitation des nanoparticules, la mesure d'un signal photoacoustique des nanoparticules, la mesure d'une radioactivité des nanoparticules, ou une combinaison de celles-ci, de préférence dans laquelle le procédé de mesure inclut une spectrométrie de masse à plasma inductif (ICP-MS) ou une spectrométrie optique à plasma inductif (ICP-OES).

19. Méthode selon l'une quelconque des revendications 1-18, dans laquelle le paramètre caractéristique est sélectionné dans le groupe consistant en : une concentration des nanoparticules, une quantité des nanoparticules, une efficacité d'élimination par clairance, une composition de ligands sur la surface de nanoparticule, un type de ligand de surface, une distribution granulométrique des nanoparticules, une charge superficielle des nanoparticules, un spectre d'absorption des nanoparticules, un spectre d'émission des nanoparticules, un spectre d'excitation des nanoparticules, un signal photoacoustique des nanoparticules, une radioactivité des nanoparticules, et une combinaison de ceux-ci.

20. Méthode selon la revendication 19, dans laquelle :
(ai) le paramètre caractéristique est la concentration des nanoparticules dans l'échantillon sanguin du sujet ;
(aii) le paramètre caractéristique témoin est la concentration témoin des nanoparticules dans l'échantillon sanguin du groupe témoin ; et
(aii) si la concentration est considérablement différente de la concentration témoin, alors il indique un dysfonctionnement ou une lésion rénal(e) ; ou
(bi) le paramètre caractéristique est l'efficacité d'élimination par clairance des nanoparticules chez le sujet ;
(bii) le paramètre caractéristique témoin est l'efficacité d'élimination par clairance témoin des nanoparticules dans le groupe témoin ; et
(biii) si l'efficacité d'élimination par clairance est considérablement différente de l'efficacité d'élimination par clairance témoin, alors il indique un dysfonctionnement ou une lésion rénal(e) ; ou
(ci) le paramètre caractéristique est le spectre d'émission des nanoparticules dans l'échantillon urinaire du sujet ;
(cii) le paramètre caractéristique témoin est le spectre d'émission témoin des nanoparticules dans l'échantillon urinaire du groupe témoin ; et
(ciii) si le spectre d'émission témoin est considérablement différent du spectre d'émission témoin, alors il indique un dysfonctionnement ou une lésion rénal(e) .

21. Méthode selon l'une quelconque des revendications 1-20, dans laquelle l'administration est à effectuer par voie intraveineuse, intrapéritonéale, sous-cutanée ou intra-artérielle.
